# FASCICULE DE BREVET EUROPEEN

(11) **EP 2 274 000 B1**
(45) Date de publication et mention de la délivrance du brevet: **08.01.2014**
(21) Numéro de dépôt: 08840967.7
(22) Date de dépôt: 16.10.2008
(51) Int. Cl.: A61K 36/71, A61K 36/63, A23L 1/30, A61P 3/10, A61P 3/04

(54) **COMPOSITION PHARMACEUTIQUE OU DIETETIQUE COMPRENANT DES EXTRAITS AQUEUX DE NIGELLA SATIVA ET D'OLEA EUROPEA ET/OU PHYLLANTUS AMARUS**
PHARMAZEUTISCHE ODER DIÄTETISCHE ZUSAMMENSETZUNG ENTHALTEND WASSEREXTRAKTE VON NIGELLA SATIVA UND OLEA EUROPEA UND/ODER PHYLLANTUS AMARUS
PHARMACEUTICAL OR DIETETIC COMPOSITION COMPRISING AN AQUEOUS EXTRACTS OF NIGELLA SATIVA AND OLEA EUROPEA AND/OR PHYLLANTUS AMARUS

(30) Priorité: 16.10.2007 FR 0758369
(43) Date de publication de la demande: 19.01.2011
(73) Titulaire: Eto, Bruno, 62000 Arras (FR)
(72) Inventeur: Eto, Bruno, 62000 Arras (FR)
(74) Mandataire: Pontet Allano & Associes
(86) Numéro de dépôt international: PCT/FR2008/051872
(87) Numéro de publication internationale: WO 2009/053652

(56) Documents cités:
- GB-A- 2 355 189
- US-A- 6 042 834
- US-A1- 2006 286 180
- ALTAN M.F.: "Effect of nigella sativa and human parathyroid hormone on bone mass and strength in diabetic rats." BIOLOGICAL TRACE LEMENT RESEARCH, vol. 116, no. 3, juin 2007 (2007-06), pages 321-328, XP002479495
- RCHID H. ET AL.: "Nigella sativa seed extracts enhance gkucose-induced insulin release from rat isolated Langerhans islets." FUNDAMENTAL AND CLINICAL PHARMACOLOGY, vol. 18, no. 5, octobre 2004 (2004-10), pages 525-529, XP002479496
- DATABASE WPI Week 1984 Thomson Scientific, London, GB; AN 1984-271992 XP002479498 "extract of leaves of olive tree used in oral treatment of diabetes mellitus." & IL 63 842 A (BABAY D.) 31 août 1984 (1984-08-31)
- GONZALEZ M ET AL: "HYPOGLYCEMIC ACTIVITY OF OLIVE LEAF" PLANTA MEDICA, THIEME, STUTTGART, DE, vol. 58, 1 janvier 1992 (1992-01-01), pages 513-515, XP009036208 ISSN: 0032-0943
- BENNANI-KABCHI N. ET AL.: "Effects of Olea europea var. oleaster leaves in hypercholesterolemic insulin-resistant rats." THERAPIE, vol. 54, no. 6, décembre 1999 (1999-12), XP009099955 London

## Description

La présente invention concerne une nouvelle application thérapeutique ou diététique du nigella sativa.

On sait que la nigella sativa, communément appelé cumin noir, fait partie de la famille des renonculacées et se présente sous la forme de graines.

Le brevet US 5 653 981 a décrit la nigella sativa sous forme de composition pharmaceutique pour traiter le cancer.

D'autres documents, comme le brevet GB 2 355 189, proposent d'utiliser des parties quelconques de nigella sativa pour stimuler la reconstitution des cellules pancréatiques, par exemple pour traiter les symptômes du diabète.

Le diabète est une maladie permanente provoquée par l'absence de production par le corps humain d'une quantité suffisante d'insuline ou par l'incapacité du corps d'utiliser efficacement l'insuline qu'il produit. Le corps a besoin d'insuline pour transformer en énergie le sucre présent dans la nourriture. En cas de diabète, le sucre reste dans le sang et finit par s'y trouver en concentrations trop élevées. Sur une longue période, de telles concentrations peuvent mener à des complications, comme des lésions aux vaisseaux sanguins et aux reins, ainsi que des problèmes de circulation.

Il existe trois types principaux de diabète : le type 1, quand le corps produit peu d'insuline ou n'en produit pas du tout. Le type 2, quand le corps produit de l'insuline sans pouvoir l'utiliser efficacement. Le diabète gestationnel, quand le corps ne peut utiliser efficacement l'insuline lors de la grossesse. Ce type de diabète disparaît après la naissance du bébé.

Chez le sujet normal, la glycémie varie entre 0.8 en fin de nuit et 1,2 g/l 1h30 après un repas soit entre 4,4 - 6,7 mmol/l. Il est possible de trouver des glycémies à jeun ou après effort entre 0,6 et 0,8g/l chez des sujets jeunes et/ou très sportif mais généralement sans aucun signe d'appel.

L'hypoglycémie est un taux anormalement bas de glucose dans le sang. Bien que l'hypoglycémie se manifeste souvent chez les personnes atteintes de diabète insulinodépendant, on la rencontre parfois chez certaines personnes en bonne santé et on l'étudie maintenant comme un trouble spécifique de l'organisme.

L'hypoglycémie provoque des symptômes nombreux et variés, et lorsqu'elle se produit chez un diabétique, elle peut avoir des conséquences graves. Dans la plupart des autres cas, l'hypoglycémie peut être contrôlée par le régime alimentaire et par un mode de vie adapté.

Le glucose constitue la principale source d'énergie de l'organisme. Pendant la digestion, le pancréas libère une hormone, le glucagon, qui transforme les glucides alimentaires en glucose, un sucre simple qui sera libéré dans le sang. Pour aider le glucose à pénétrer dans les cellules, le pancréas sécrète une deuxième hormone, l'insuline. Sans insuline, le glucose s'accumule dans le sang et ne procure pas l'énergie nécessaire aux cellules. On parle alors d'hyperglycémie et cette condition mène souvent au diabète. Si, au contraire, il y a trop d'insuline, le glucose ne suffit pas à la tâche et les cellules sont privées d'énergie. Le taux de sucre baisse et les symptômes de l'hypoglycémie peuvent se manifester.

L'organisation mondiale de la santé (OMS) a défini deux principaux critères de diagnostic du diabète :
1) Le dosage de la glycémie plasmatique à jeun en laboratoire après 8 à 12 heures de jeûne. Si la glycémie à jeun est inférieure à 1,10 g/l, le sujet est "normal". Si la glycémie à jeun est supérieure à 1,26 g/l, le sujet est diabétique. Si la glycémie à jeun est comprise entre 1,10 et 1,26 g/l, le sujet est classé comme atteint d'hyperglycémie à jeun (intolérance au glucose). Ces sujets ne sont pas diabétiques mais sont à haut risque de le devenir en l'absence de mesures hygiéno-diététiques.
   Pour les valeurs limites, il est indispensable, avant de poser un diagnostic, de confirmer les premiers résultats en faisant faire un nouveau dosage quelques semaines plus tard.
2) Le dosage de la glycémie mesurée deux heures après l'administration par voie orale d'une dose d'épreuve de 75g de glucose (glycémie post prandiale).
Le problème dans cette méthode est que les valeurs limites fixées (deux heures après) proviennent d'études épidémiologiques portant sur un nombre d'individus relativement faible.

Le dépistage du diabète doit être ciblé c'est à dire réservé aux sujets à haut risque d'être ou de devenir diabétiques de type II. Le but est donc de repérer les sujets à risque. Le diabète de type II survient habituellement après l'âge de 40 ans. C'est donc chez des sujets ayant atteint ou dépassé cet âge qu'il doit être recherché.

Doivent aussi être dépistés, les sujets ayant un membre de leur famille atteint de diabète, et les sujets hypertendus sous traitement médicamenteux fréquemment en surpoids.

La prise en charge d'un patient diabétique est difficile car c'est une longue maladie. Son traitement quotidien altère profondément la qualité de vie du diabétique. Il faut qu'il soit global, dirigé contre tous les facteurs de risque, pour prévenir les complications micro et macro vasculaires de la maladie.

Les outils de prise en charge sont les principes hygiéno-diététiques à savoir :
- réduction de l'excès pondéral: "des régimes modérément restrictifs entraînent de meilleurs résultats à long terme et induisent moins d'effets secondaires que les restrictions alimentaires sévères" (niveau de preuve C),
- répartition des nutriments: concernent à la fois les patients en surpoids et les patients à poids normal (accord d'expert). La répartition recommandée entre les apports respectifs des glucides et des lipides alimentaires doit tenir compte du profil des habitudes alimentaires du patient (niveau preuve C),
- l'observance diététique: il est recommandé pour l'améliorer d'assurer un suivi diététique régulier et de coupler aux conseils diététiques des conseils d'activité physique (accord d'expert),
- l'activité physique : la pratique d'une activité sportive est recommandée dans les cas où elles sont applicables (niveau de preuve C).

La stratégie de prise en charge des patients est la suivante :
Quand par exemple un diabète de type II est diagnostiqué, on a recours tout d'abord à un traitement non pharmacologique. Pendant 4 à 6 mois, le patient diabétique doit respecter les règles hygiéno-diététiques citées ci-dessus : régime réduisant les aliments gras, les boissons sucrées et alcoolisées, la reprise d'une activité physique régulière pendant 30 à 45 minutes, 3 à 4 jours par semaine. Ceci permettra une perte de poids et une significative réduction des valeurs glycémiques, tensionnelles et lipidiques. En cas d'échec après 4 à 6 mois, il faut alors opter pour un traitement médicamenteux.

La deuxième étape consiste à la monothérapie orale. On dispose actuellement de médicaments antidiabétiques actifs par voie orale à action hypoglycémiante. Les hypoglycémiantes per os les plus connus sont:
- Biguanides : ils améliorent la sensibilité à l'insuline.
- Sulfamides hypoglycémiants : ils stimulent la sécrétion d'insuline et ont une activité hypoglycémiante plus marquée.
- Inhibiteurs des α-glucosidases: ils ont une activité hypoglycémiante plus faible.
- Insulino-secrétagogues (glinidines): ils sont surtout actifs sur l'hyperglycémie post prandiale.

Ces médicaments utilisés en monothérapie permettent d'obtenir un contrôle glycémique correct qui est évalué tous les 4 mois par un dosage de l'hémoglobine glyquée (HbA1c).
- l'objectif optimal à atteindre est une valeur d'HbA1c inférieure ou égale à 6,5%
- lorsque l'HbA1c se situe entre 6,6 et 8% sur deux contrôles successifs, une modification du traitement peut-être envisagée en fonction de l'appréciation par le clinicien du rapport avantages/inconvénients du changement de traitement envisagé
- lorsque l'HbA1c est supérieure à 8% sur deux contrôles successifs, une modification du traitement est recommandée (accord professionnel).

La troisième étape est la bithérapie orale. En cas d'échec primaire ou secondaire de la monothérapie orale initiale, il est recommandé de prescrire une bithérapie orale c'est à dire d'associer entre elles deux classes d'hypoglycémiants.

La quatrième étape est l'insulinothérapie. Si un diabétique de type II demeure mal équilibré, il faut passer à un traitement par l'insuline. L'insulinothérapie permet d'obtenir une amélioration nette du contrôle glycémique. Si ce n'est pas le cas, on peut associer à l'insulinothérapie la prescription de biguanides qui en potentialisent l'action.

Les principales complications dues au diabète sont nombreuses. Parmi ces complications on peut citer:
- Complications aiguës du diabète insulinodépendant. Ces sont des urgences métaboliques (malaise voire comas) par hyperglycémie et acidocétose (insuline non prescrite ou insuffisamment dosée) mais aussi par hypoglycémie résultant de l'administration de quantités inadaptées d'insuline.
- Complications chroniques et dégénératives des diabètes. Parmi lesquelles, la rétinopathie diabétique qui est la principale cause de cécité et de troubles visuels, l'insuffisance rénale liée à la gravité et à la durée de la maladie, la cardiopathie responsable dans les pays industrialisés de 75 % des décès, les neuropathies diabétiques qui sont probablement les complications les plus courantes du diabète. Selon certaines études, 50 % des diabétiques voir plus en souffrent à des degrés divers, l'ulcération des pieds et l'amputation.

La prise de poids est aussi devenue un problème majeur de la société de consommation. Toutes les études épidémiologiques montrent que le nombre de personnes qui vivent avec le surpoids augmente régulièrement dans les pays industrialisés ainsi que dans les pays en voie de développement. Cette prise de poids est surtout la conséquence d'une mauvaise nutrition. Une alimentation riche en sucre plus particulièrement le glucose et le fructose est souvent l'un des déclencheurs de la prise de poids.

### Objectifs

Le but de l'invention est de proposer une nouvelle composition pharmaceutique et/ou diététique qui agit comme un bloqueur de l'absorption intestinale des sucres et de ses dérivés, ainsi que comme traitement ou prévention contre les affections qui y sont liées.

Le déposant a constaté, de façon surprenante, qu'un extrait aqueux de nigella sativa était utilisable en tant que composition pharmaceutique ou diététique pour inhiber ou bloquer l'absorption intestinale du sucre.

L'invention a ainsi pour objet une composition pharmaceutique et/ou diététique renfermant au moins un extrait aqueux de nigella sativa associé notamment avec un extrait aqueux de feuilles d'olea europaea.

Le terme extrait aqueux est ici à prendre au sens d'un extrait obtenu par l'eau mais non par l'alcool ou par une huile.

La présente invention est différente de toutes les autres inventions utilisant les extraits des graines de nigelle (nigella sativa) comme composition pharmaceutique utilisée pour stimuler les fonctions immunitaires (brevet US N° 5653981). Plusieurs compositions pharmaceutiques contenant plusieurs plantes en association avec la nigelle ont été utilisées. Mais toutes ces formulations pharmaceutiques sont faites soit avec les extraits totaux de nigelle, soit les extraits organiques ou huileux. Aucune de ces compositions ne sont faites à partir des seuls extraits aqueux (voir également les brevets US N°6042834 et US N°6218434).

Plusieurs articles scientifiques décrivent aussi l'utilisation des extraits huileux ou totaux des graines de nigelle comme antidiabétique. Il est de l'article de Otoom et al (J Herb Pharmacother. 2006, 6(2) : 31-41). Il en est de même pour l'article de Fararh KM (Res Vet Sci. 2004 Oct, 77(2) :123-9), de El-Dakhakhny M (Planta Med. 2002 may 68(5) :465-6), de Zubaida A (Annals of Saudi Medicine, vol 21, N°3-4, 2001).

### Synergie avec une deuxième plante

La composition selon l'invention peut renfermer en outre un extrait aqueux d'au moins une autre plante produisant une synergie avec nigella sativa, par exemple de l'olea europaea dans l'une ou l'autre de ses sous espèces (europaea, africana, cerasiformis, cuspidata, guanchica, laperrinei, maroccana).

Ainsi qu'il est montré ci-après, cette combinaison de Nigella Sativa et de Olea Europaea présente une synergie produisant de meilleurs résultats que l'addition des résultats obtenus individuellement par chacune de ces plantes. Cette synergie est probablement due à la présence, dans les extraits aqueux de ces deux plantes, de composés ayant une action commune ou similaire quand bien même ils seraient de natures différentes.

Dans le cadre de l'invention, il est aussi possible de remplacer l'extrait de feuilles d'olea europaea, en tout ou partie, par d'autres plantes fournissant de tels composés à action commune ou similaire.

L'invention propose ainsi une composition comprenant, outre l'extrait aqueux de graine de Nigella Sativa, un extrait aqueux d'au moins une deuxième plante issue du groupe suivant :
Olea europaea (feuilles) ou
Phyllantus amarus (feuilles).

De préférence, l'extrait aqueux selon l'invention, referme entre 1% et 99% en poids de nigella sativa par rapport au poids total de la composition ou du médicament.

Cette composition ou ce médicament peut renfermer en outre de 0,001% à 50%, en poids d'extrait aqueux d'olea europaea ou de la deuxième plante par rapport au poids total de la composition ou du médicament, et de préférence de 0,001% à 30%.

### Formes galéniques

Selon l'invention, la composition peut être présentée sous forme de comprimé ou gélule et comprend entre 30% et 50% en poids de Nigella Sativa par rapport au poids total de cette composition.

Plus particulièrement, cette composition peut comprendre entre 10% et 30% en poids de la deuxième plante, Olea Europaea pour la BioDiabétine^{®}, par rapport au poids de Nigella Sativa dans ladite composition. Une proportion préférée en poids d'extrait pour la deuxième plante est de 20% par rapport à l'extrait de Nigella Sativa.

### Applications

Cette composition peut être utilisée notamment :
- en tant que médicament ou composition diététique pour lutter contre la prise de poids, voire l'obésité,
- en tant que moyen ou outil pour inhiber l'absorption des sucres au cours d'études expérimentales, fondamentales ou appliquées.

Cependant, l'application préférée de l'extrait aqueux de nigella sativa est le traitement et la prévention du diabète.

La composition pharmaceutique selon l'invention peut être présentée sous forme administrable par voie orale, anale ou intraveineuse.

D'autres particularités et avantages de l'invention ressortiront de la description détaillée d'un mode de mise en oeuvre nullement limitatif, et des figures annexées dans lesquelles :

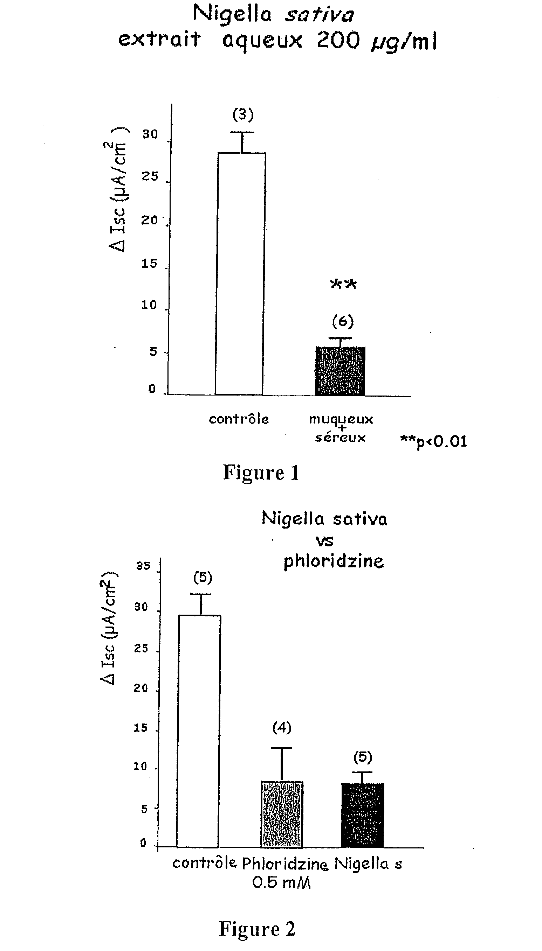

- la figure 1 est un graphique d'essais illustrant l'effet de l'extrait aqueux de Nigella Sativa sur le courant de court-circuit (Isc). Isc reflète dans cette expérience le transport du glucose à travers la barrière intestinale. Ces essais montrent que l'extrait aqueux de la nigelle sativa, utilisée à une concentration de 200µg/ml inhibe l'absorption intestinale du glucose, lorsqu'elle est introduite dans le côté muqueux et séreux, avec une probabilité supérieure à 99% (** : p<0,01) ;
- la figure 2 est un graphique d'essais qui montre que l'extrait aqueux de la nigelle sativa, utilisée à une concentration de 200µg/ml inhibe l'absorption intestinale du glucose, avec une même amplitude que lorsqu'on utilise 0,5 mg/ml de phloridzine qui est un puissant inhibiteur de l'absorption intestinale du glucose au niveau de l'intestin grêle ;

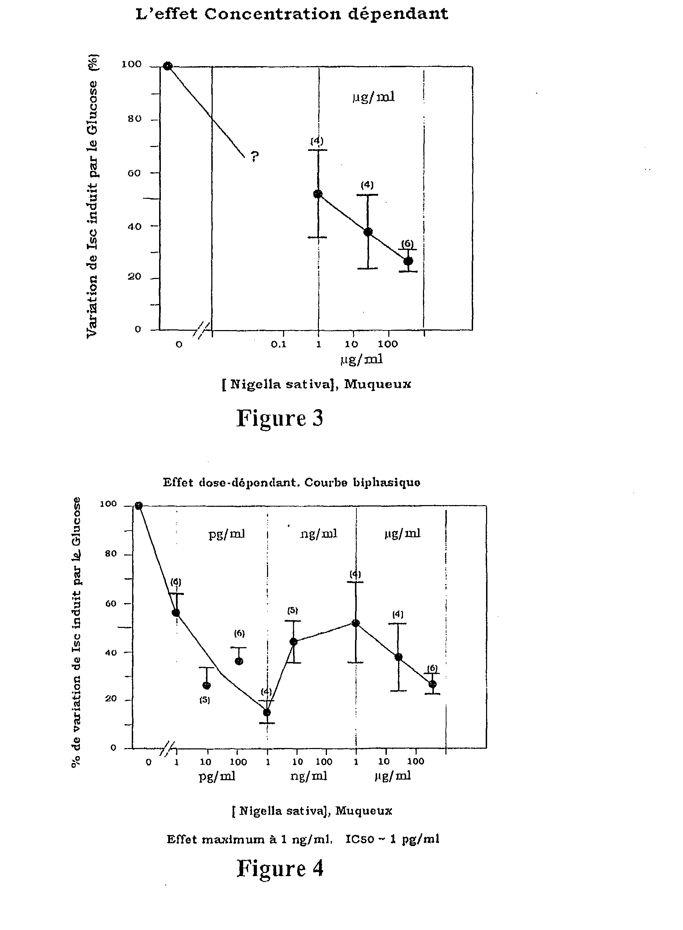

- la figure 3 est un graphique d'essais qui montre que l'effet de l'extrait aqueux de la nigelle sativa, sur l'inhibition de l'absorption du glucose, est dépendant de la concentration ;
- la figure 4 est un graphique d'essais qui montre que l'effet dose-dépendant du principe actif de la nigella sativa est biphasique ;

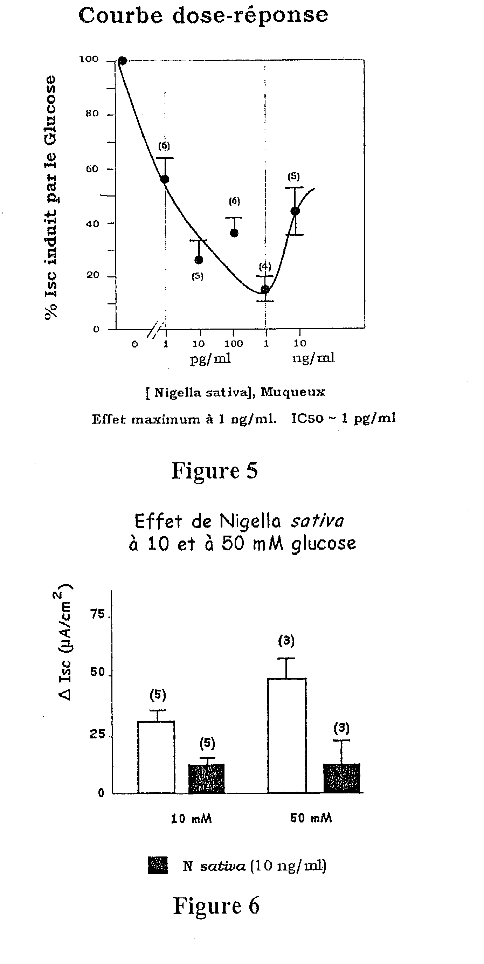

- la figure 5 est un graphique d'essais illustrant la mise en valeur de la détermination de la concentration inhibitrice à 50% de l'effet maximum du principe actif sur l'absorption intestinale du glucose. L'inhibition maximale est obtenue avec une concentration de 1ng/ml. L'inhibition de 50 % (IC50) de l'absorption intestinale du glucose est obtenue avec une concentration de 1pg/ml (IC50 = 1pg/ml) ;
- la figure 6 est un graphique d'essais illustrant l'effet comparé de l'extrait aqueux de Nigella Sativa pour deux dosages différents de glucose à savoir 10mM et 50mM. Ces essais montrent que l'extrait aqueux de la nigelle sativa, utilisée à une concentration de 10ng/ml, inhibe avec la même amplitude l'effet de 10 mM ou de 50 mM de glucose. Cette figure montre que l'effet inhibiteur de la nigelle concerne plusieurs transporteurs différents de glucose ;

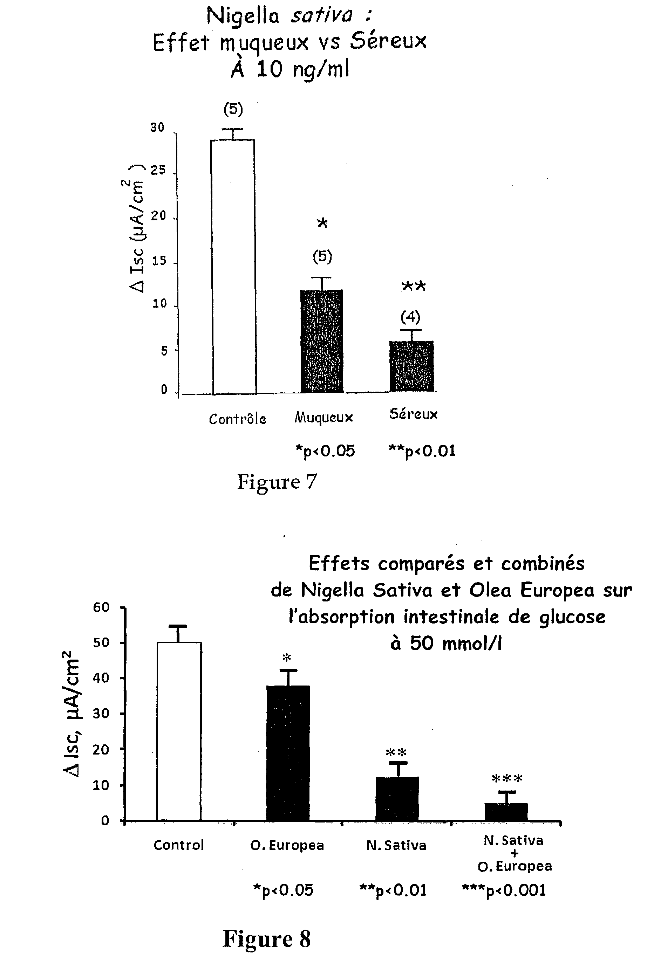

- la figure 7 est un graphique d'essais illustrant l'effet comparé de l'extrait aqueux de Nigella Sativa, selon qu'on l'introduit du côté muqueux ou du côté séreux du morceau d'intestin dans le dispositif de chambre d'Ussing. Ces essais montrent qu'il existe un effet inhibiteur de l'extrait aqueux de Nigella Sativa, utilisée à une concentration de 10ng/ml. Ils montrent aussi que cet effet est plus important du côté séreux que du côté muqueux. Ces résultats sont un indicateur de l'efficacité lors d'une administration par voie intraveineuse (pour le côté séreux) et par voie orale ou anale (pour le côté muqueux). Les valeurs statistiques de probabilité sont respectivement de 99% (côté séreux, ** : p<0,01) et de 95% (côté muqueux, * : p<0,05) ;
- la figure 8 est un graphique d'essais illustrant les effets comparés d'extraits aqueux de Nigella Sativa seul, de Olea Europaea seul, et de l'effet combiné des deux extraits. Ces essais montrent que l'effet combiné de l'extrait de Nigella Sativa avec l'extrait de Olea Europaea est plus important que le cumul des effets de ces deux plantes testées séparément, ce qui indique bien une synergie obtenue par cette combinaison.

### Mise en oeuvre de l'invention : la BioDiabétine^{®}

L'invention est actuellement mise en oeuvre sous le nom de « BioDiabétine^{®} » ou « BioD^{®} », dont le principe actif est une combinaison de l'extrait aqueux de la nigelle sativa ou cumin noir et de l'extrait de feuille de l'olivier d'Europe (Olea Europaea).

A titre d'exemple non limitatif mais portant sur un modèle de réalisation de la formulation, le principe actif de la BioD^{®} peut être réalisé avec une concentration de l'extrait aqueux de graines de nigelle sativa qui varie entre 0,1 à 99% et une concentration de l'extrait aqueux des feuilles de l'olivier de 0,1 à 30% voire 50%.

La BioDiabétine^{®} sous forme de comprimé présente les formulations suivantes :

| **Comprimé de** | **990mg** | | | **665mg** | | |
|---|---|---|---|---|---|---|
| **Désignation des matières** | Formule unitaire (mg) | % total | %/N.S. | Formule unitaire (mg) | % total | %/N.S. |
| Nigella Sativa (extrait sec) | 455 | 46,0 | x | 227,5 | 34,2 | x |
| Olea Europaea (extrait sec) | 91 | 9,2 | 0,20 | 45,5 | 6,8 | 0,20 |
| cellulose microcristalline (Vivapur 102) | 234 | 23,6 | | 217 | 32,6 | |
| amidon de mais prégélatinisé (Lycatab) | 75 | 7,6 | | 60 | 9,0 | |
| croscarmellose sodique | 110 | 11,1 | | 95 | 14,3 | |
| silice colloïdale anhydre (Aérosil 200) | 5 | 0,5 | | 3 | 0,5 | |
| acide stéarique | 20 | 2,0 | | 7 | 1,1 | |
| **TOTAL** | 990 | 990 | | 665 | 665 | |

Dans ses différentes formulations, et par exemple celles mentionnées ci-dessus, la composition peut aussi être présentée sous forme de gélules ou de poudre pour dilution ou sirop. Cette poudre peut ne contenir que les principes actifs, et est obtenue par exemple par atomisation, ou séchage, ou lyophilisation à partir de l'extrait aqueux.

### Exemple de préparation de l'extrait aqueux

Les extraits des graines de nigelle séchés sont broyés de préférence à froid. La poudre est mélangée à l'eau puis laissée à macération. La macération est ensuite filtrée puis atomisée pour obtenir une poudre fine. Le produit obtenu après filtration de la macération peut aussi être séché soit à l'étuve, soit au four, soit lyophilisé.

Le même procédé est utilisé pour obtenir la poudre des extraits aqueux de la deuxième plante, ici des feuilles de l'Olivier. Les deux poudres sont mélangées pour obtenir le principe actif de la BioD^{®}.

L'homme du métier connait et utilise différents excipients comme liants ou lubrifiants pour fabriquer les poudres, les granulés ou autres préparations. Des exemples d'excipients qui peuvent être ainsi utilisés selon l'invention sont : le lactose, l'amidon, la dextrine, le phosphate de calcium, le carbonate de calcium, le silicate d'aluminium naturel ou synthétique, l'oxyde de magnésium, l'hydroxyde d'aluminium hydraté, le stéarate de magnésium, le bicarbonate de sodium. D'autres excipients non cités ci-dessus mais qui conviennent également sont décrits dans le « Remington's Pharmaceutical Sciences » par E.W. Martin, et peuvent être utilisés selon l'invention.

Les comprimés ou autres formes galéniques sont alors réalisés selon des méthodes connues par l'homme du métier.

### Essais du principe actif

Il a été trouvé de manière inattendue que l'administration par voie orale (per os) d'une quantité efficace du mélange d'un extrait aqueux de nigella sativa provoquait un blocage de l'absorption intestinale des sucres.

Ces résultats ont été confirmés avec les études in vitro sur un morceau d'intestin en chambre de perméation d'Ussing, qui ont montré que la composition selon la présente invention inhibe l'absorption intestinale du sucre.

La description ci-après montre à l'aide des chambres de perméation d'Ussing l'effet inhibiteur de la composition selon l'invention (Nigella Sativa), sur l'absorption intestinale des sucres chez le rat.

Les résultats montrent que la BioD^{®} inhibe l'absorption intestinale du glucose. Cette inhibition du glucose est dépendante de la dose et l'effet maximum est obtenu avec une concentration de 1 nanogramme d'extrait par millilitre.

### Matériels et méthodes utilisés

### Produits chimiques

La Bumétanide, la Phloridzine et le glucose ont été achetés chez Sigma (St Quentin-Fallavier, France). La Biodiabétine^{®} ou BioD^{®} a été préparé par le laboratoire de Biotechnologie de la société Titis Business Corporation France (T.B.C).

### Préparation de tissus

Les rats Sprague-Dawley adultes pesant entre 180 et 250 g ont été obtenus d'Iffa Credo Iffa (St Germain s / Arbresle, France) et ont été nourris par les aliments standard de laboratoire (UAR, Villemoisson s / Orge, France) jusqu'à la réalisation de ces études. La nourriture a été retirée 18 h avant les expériences, mais les animaux avaient l'accès libre à eau potable. Pour les études de l'électrophysiologie, les animaux ont été tués par la dislocation cervicale après anesthésie et segments d'intestin d'animaux à jeun ont été enlevés et rincés de contenu intestinal avec solution du Ringer frais. Les estomacs de tous les animaux ont été trouvés vides. Les morceaux de tissus ont été ensuite ouverts le long de la bordure mésentérique ont été montés bien aplatis entre les deux demi chambres Ussing acryliques (Titis Business Corporation -TBC France).

### Etudes du courant de Court-circuit en chambres Ussing

La solution du Ringer isotonique est utilisée dans les expériences est composée de (en mM) 115 NaCl, 25 NaHCO₃, 1.2 MgCl₂, 1.2 CaCl₂, 2.4 K₂HPO₄ et 0.4 KH₂PO₄. Le pH était 7.40 à 37°C.

Une préparation intestinale est montée entre deux demi chambres en plexiglas déterminant un compartiment muqueux et un compartiment séreux.

Ces demi-chambres, dont l'ouverture est appliquée contre la surface exposée du tissu intestinal, sont perforées en compartiments contenant le liquide de Ringer qui est une solution tamponnée à pH = 7,6 tempérée à 37°C. Le pH du liquide de Ringer et sa saturation en oxygène sont maintenus constamment par un bullage permanent de carbogène, mélange gazeux composé d'oxygène et de dioxyde de carbone dans les proportions 95% de O₂ et 5% de CO₂. Le bullage assure également le brassage permanent du milieu d'incubation dans les deux compartiments.

Le potentiel électrique transépithélial reflétant l'asymétrie de charges électriques entre le coté muqueux et séreux de la membrane, a été mesuré par les électrodes de calomel reliées par des ponts d'agaroses plongés dans une solution de KCI de 3 M dans 4% (w/v). Ces ponts ont été placés sur les deux côtés du tissu et sont adaptés à chaque demi chambre. Le tout est relié à un voltmètre à haute impédance.

La différence de potentiel a été court-circuitée par un courant de court-circuit (Icc ou Isc) via des ponts d'agaroses à 3M de KCI placés dans chaque réservoir, adapté aux électrodes Ag-AgCl, le tout étant relié à un système de la voltage-Clamp (Titis Business Corporation ou TBC France). Isc délivré et corrigé pour résistance fluide, a été enregistré de façon continue sur un ordinateur à l'aide d'un logiciel Daqsoft (TBC France). Isc représente la somme des flux d'ions nets transportés à travers l'épithélium en absence d'un gradient électrochimique (principalement Na+, Cl⁻₂ et HCO⁻₃).

### Effet de l'extrait aqueux de Nigella Sativa sur l'inhibition du transport du glucose

Les paramètres électriques indiquent le degré de perméabilité de la membrane intestinale, et donc correspondent à la capacité des transporteurs du glucose à transporter le glucose à travers cette membrane intestinale.

Après la stabilisation des paramètres électriques des tissus, l'extrait aqueux de Nigella Sativa a été introduit à différentes concentrations dans les compartiments séreux et muqueux des préparations, alors que le glucose n'a été introduit que dans le compartiment muqueux. Pour éviter la pression osmotique engendrée par l'introduction du D-Glucose dans le compartiment muqueux, les mêmes concentrations de mannitol ont été ajoutées dans le côté séreux de la préparation. Les variations des paramètres électriques sont ensuite relevées puis enregistrés sur l'ordinateur.

### Statistiques

Les résultats, montrés sur les figures annexées, sont rapportés en moyenne (M) de mesures plus l'écart type de ma moyenne (M ± ETM). Toutes les déterminations ont été faites avec une moyenne d'au moins de 6 morceaux de tissu (n). L'analyse de la variance a été effectuée d'après la procédure du modèle linéaire suivi du test de Duncan grâce au logiciel SAS (SAS Institute, Cary, NC,).

### Résultats

Ainsi que détaillé plus haut en référence aux figures, les résultats de ces essais confirment l'action de la composition selon l'invention dans sa version Nigella Sativa seule.

La synergie obtenue par la combinaison d'extraits aqueux de Nigella Sativa et Olea Europaea est montrée par les essais illustrés en figure 8, dont les chiffres sont statistiquement significatifs (valeurs de « p ») et sont présentés dans le tableau suivant :

| | ΔIsc, µA/cm² | p |
|---|---|---|
| Control | 50 | |
| O. Europaea | 38 | * : p < 0,05 |
| N. Sativa | 12,5 | ** : p < 0,01 |
| N. Sativa + O. Europaea | 5,2 | *** : p < 0,001 |

L'association avec Olea Europaea présente ainsi une efficacité supérieure à celle de Nigella Sativa seule telle que montrée par les essais illustrés aux figures 1 à 7.

Bien sûr, l'invention n'est pas limitée aux exemples qui viennent d'être décrits et de nombreux aménagements peuvent être apportés à ces exemples sans sortir du cadre de l'invention.

## Revendications

1. Composition, comprenant un extrait aqueux de Nigella Sativa et un extrait aqueux d'au moins une deuxième plante issue du groupe suivant : feuilles de Olea europaea et feuilles de Phyllantus amarus.

2. Composition selon la revendication 1, pour son utilisation en tant que médicament.

3. Composition selon la revendication 1, **caractérisée en ce qu'**elle comprend entre 1% et 99% en poids de Nigella Sativa par rapport au poids total de ladite composition.

4. Composition selon l'une quelconque des revendications 1 et 3, **caractérisée en ce qu'**elle est présentée sous forme de comprimé ou gélule et comprend entre 30% et 50% en poids de Nigella Sativa par rapport au poids total de ladite composition.

5. Composition selon l'une quelconque des revendications 1 et 3 à 4, **caractérisée en ce qu'**elle comprend de 0,001% à 50% en poids de Olea Europaea par rapport au poids total de ladite composition.

6. Composition selon la revendication 5, **caractérisée en ce qu'**elle comprend de 0,001% à 30% en poids de Olea Europaea par rapport au poids total de ladite composition.

7. Composition selon la revendication 6, **caractérisée en ce qu'**elle est présentée sous forme de comprimé ou gélule et comprend entre 10% et 30% en poids de Olea Europaea par rapport au poids de Nigella Sativa dans ladite composition.

8. Composition selon l'une quelconque des revendications 1 à 3 ou 7, **caractérisée en ce qu'**elle est présentée sous forme de poudre pour dilution ou sirop, obtenue par atomisation ou séchage ou lyophilisation à partir de l'extrait aqueux.

9. Composition selon la revendication 1, pour son utilisation en tant que médicament contre le diabète.

10. Composition selon la revendication 1, pour son utilisation en tant que médicament contre l'obésité.

11. Utilisation non-thérapeutique en tant que produit diététique pour lutter contre la prise de poids d'une composition selon l'une quelconque des revendications 1 et 3 à 8.

## Patentansprüche

1. Zusammensetzung, umfassend einen wässrigen Extrakt aus Nigella sativa und einen wässrigen Extrakt aus wenigstens einer zweiten Pflanze, die aus folgender Gruppe stammt: Blätter von Olea europaea und Blätter von Phyllantus amarus.

2. Zusammensetzung nach Anspruch 1, für ihre Verwendung als Medikament.

3. Zusammensetzung nach Anspruch 1, **dadurch gekennzeichnet, dass** sie bezogen auf das Gesamtgewicht der Zusammensetzung zwischen 1 Gew.-% und 99 Gew.-% Nigella sativa umfasst.

4. Zusammensetzung nach einem der Ansprüche 1 und 3, **dadurch gekennzeichnet, dass** sie in Form einer Tablette oder Kapsel angeboten wird und bezogen auf das Gesamtgewicht der Zusammensetzung zwischen 30 Gew.-% und 50 Gew.-% Nigella sativa umfasst.

5. Zusammensetzung nach einem der Ansprüche 1 und 3 bis 4, **dadurch gekennzeichnet, dass** sie bezogen auf das Gesamtgewicht der Zusammensetzung 0,001 Gew.-% bis 50 Gew.-% Olea europaea umfasst.

6. Zusammensetzung nach Anspruch 5, **dadurch gekennzeichnet, dass** sie bezogen auf das Gesamtgewicht der Zusammensetzung 0,001 Gew.-% bis 30 Gew.-% Olea europaea umfasst.

7. Zusammensetzung nach Anspruch 6, **dadurch gekennzeichnet, dass** sie in Form einer Tablette oder Kapsel angeboten wird und, bezogen auf das Gewicht von Nigella sativa in der Zusammensetzung, zwischen 10 Gew.-% und 30 Gew.-% Olea europaea umfasst.

8. Zusammensetzung nach einem der Ansprüche 1 bis 3 oder 7, **dadurch gekennzeichnet, dass** sie in Form eines Pulvers zum Verdünnen oder zur Herstellung eines Sirups angeboten wird, das durch Zerstäuben oder Trocknen oder Lyophilisieren anhand des wässrigen Extrakts erhalten wird.

9. Zusammensetzung nach Anspruch 1, für ihre Verwendung als Medikament gegen Diabetes.

10. Zusammensetzung nach Anspruch 1, für ihre Verwendung als Medikament gegen Fettleibigkeit.

11. Nicht-therapeutische Verwendung einer Zusammensetzung nach einem der Ansprüche 1 und 3 bis 8, als diätetisches Produkt für den Kampf gegen Gewichtszunahme.

## Claims

1. Composition comprising an aqueous extract of *Nigella sativa* and an aqueous extract of at least one second plant originating from the following group: leaves of *Olea europaea* and leaves of *Phyllanthus amarus.*

2. Composition according to claim 1, for its use as a medicinal product.

3. Composition according to claim 1, **characterized in that** it comprises between 1 % and 99% by weight of *Nigella sativa* with respect to the total weight of said composition.

4. Composition according to any one of claims 1 and 3, **characterized in that** it is presented in the form of a tablet or capsule and comprises between 30% and 50% by weight of *Nigella sativa* with respect to the total weight of said composition.

5. Composition according to any one of claims 1 and 3 to 4, **characterized in that** it comprises from 0.001 % to 50% by weight of *Olea europaea* with respect to the total weight of said composition.

6. Composition according to claim 5, **characterized in that** it comprises from 0.001 % to 30% by weight of *Olea europaea* with respect to the total weight of said composition.

7. Composition according to claim 6, **characterized in that** it is presented in the form of a tablet or capsule and comprises between 10% and 30% by weight of *Olea europaea* with respect to the weight of *Nigella sativa* in said composition.

8. Composition according to any one of claims 1 to 3 or 7, **characterized in that** it is presented in the form of powder for dilution or syrup, obtained by atomization or drying or lyophilization from the aqueous extract.

9. Composition according to claim 1, for its use as medicinal product against diabetes.

10. Composition according to claim 1, for its use as medicinal product against obesity.

11. Non-therapeutic use of a composition according to any one of claims 1 and 3 to 8 as a dietetic product for combating weight gain.
